**Europäisches Patentamt**

**European Patent Office**

**Office européen des brevets**

(19)

(11) **EP 1 426 024 A1**

(12) **EUROPÄISCHE PATENTANMELDUNG**

| | |
|---|---|
| (43) Veröffentlichungstag:<br>**09.06.2004 Patentblatt 2004/24** | (51) Int Cl.⁷: **A61F 7/08** |

(21) Anmeldenummer: **03023041.1**

(22) Anmeldetag: **13.10.2003**

| | |
|---|---|
| (84) Benannte Vertragsstaaten:<br>**AT BE BG CH CY CZ DE DK EE ES FI FR GB GR HU IE IT LI LU MC NL PT RO SE SI SK TR**<br>Benannte Erstreckungsstaaten:<br>**AL LT LV MK**<br><br>(30) Priorität: **25.10.2002 DE 10249853**<br><br>(71) Anmelder: **BIONICS PHARMA GMBH**<br>**82031 Grünwald (DE)** | (72) Erfinder: **Liedtke, Rainer K., Dr.**<br>**82027 Grünwald (DE)**<br><br>(74) Vertreter:<br>**Leson, Thomas Johannes Alois, Dipl.-Ing.**<br>**Tiedtke-Bühling-Kinne & Partner GbR,**<br>**TBK-Patent,**<br>**Bavariaring 4**<br>**80336 München (DE)** |

(54) **Pflasterartige Chip-Systeme zur thermodynamischen Kontrolle topisch dermaler und transdermaler Systeme**

(57) Die Erfindung betrifft pflasterartige Chip-Systeme (4) zur thermodynamischen Kontrolle (7) topisch dermaler und transdermaler Systeme, insbesondere zur Verbesserung von Effizienz und Sicherheit dermaler und transdermaler Therapien und Diagnosen. Informationstechnisch repräsentieren die Systeme komplexe technische Vorrichtungen, die gegenüber üblichen passiven Systemen durch programmierbare wie auch individuelle Steuerung kontrollierte und intelligente Systeme darstellen (7). Ihre Implementierung in passive dermale oder transdermale therapeutische Systeme erfordert keine technischen Eingriffe in den bestehenden Aufbau solcher Systeme. Im Bereich nicht-invasiver und mikro-invasiver dermaler und transdermaler Diagnostik eröffnen die pflasterartigen Chip-Systeme zudem neue Anwendungsmöglichkeiten.

**Abbildung 3**

EP 1 426 024 A1

**Beschreibung**

Die Erfindung betrifft pflasterartige Chip-Systeme zur thermodynamischen Kontrolle topisch dermaler und transdermaler Systeme, insbesondere zur Verbesserung der Effizienz und Sicherheit topisch dermaler und transdermaler Therapien und Diagnosen.

Es ist bekannt ist, daß Anwendungen physikalischer Effekte wie auch chemischer Effekte auf und durch die Haut, zahlreiche bedeutsame Vorteile ermöglichen können.

Dermal und transdermal physikalische Effekte sind beispielsweise Anwendungen thermischer oder elektrischer Reize auf die Hautoberfläche, auf einzelne oder mehrere Hautschichten und/oder auf Haut-Anhangsgewebe. Bei thermisch therapeutischen Effekten sind, neben deren medizinisch relaxierenden und schmerzmildernden Wirkungen auf neuromuskuläre Organe und Systeme beispielsweise auch solche Art Anwendungen zu nennen wie sie mittels sogenannter regionaler Hyperthermie zur Behandlung und Sensibilisierung von Tumorgeweben stattfinden.

Zu den neueren therapeutisch chemischen Anwendungen zählen sogenannte transdermale Systeme, spezielle technische Pflastersysteme mit verschieden ausgestalteten Arzneireservoirs, aus denen Arzneistoffe kontinuierlich in die Haut freigesetzt werden und die von dort in den Kreislauf wandern. Ebenso sind dies aber auch die schon länger eingestzten halbfesten wirkstoffhaltigen pharmazeutischen Formulierungen, beispielsweise Salben, Gele, Cremes, die zur Resorption auf die Haut aufgebracht werden. Im Rahmen der transdermalen Pflastersysteme gibt es bereits einige Stoffe, die hiermit zur systemischen Therapie angewendet werden. Dazu gehören beispielweise Steroidhormone zur Hormon-Substitution bei Beschwerden der Wechseljahres als auch solche zur Kontrazeption, Nitroglycerin bei Angina Pectoris, Nikotin zur Raucherentwöhnung, Scopolamin bei Reisebeschwerden mit Vertigo, sowie die analgetischen Stoffe Fentanyl und Buprenorphin zur Therapie schwerer Schmerzzustände. Im Bereich der pharmazeutisch halbfesten Formen existieren sehr zahlreiche Formulierungen für unterschiedliche sowohl topische wie auch systemische

Anwendungsziele, beispielsweise solche zur Linderung lokaler Schmerzen oder neuromuskulärer Beschwerden, sowie Formulierungen zur lokalen Beeinflussung traumatischer oder degenerativer Hautschäden.

Zum Bereich dermaler therapeutischer Systeme zählen aber beispielsweise auch solche technischen Vorrichtungen bei denen pharmazeutisch feste oder halbfeste Formulierungen, die mittels invasiver Massnahmen, beispielsweise durch Implantation oder Injektion, in das Gewebe unter der Hautoberfläche eingebracht werden, und die dann von diesem Ort als Reservoirs über längere Zeiträume ihre Wirkstoffe kontinuierlich freisetzen. Dazu gehören technisch beispielsweise Kristallsuspensionen, kolloidisperse Formulierungen oder Depotformulierungen aus biologisch kompatiblen Stoffen, die enzymatisch erodiert werden können, und die beispielsweise Schmerzmittel oder verschiedene Hormone enthalten können.

Als dermale oder transdermale diagnostische Systeme lassen sich solche technische Vorrichtungen einordnen, die auf und/oder in die Haut gebracht werden und mit denen aus der Haut oder Hautschichten eine Information zur Körperbeschaffenheit erzielt werden kann. Dazu gehört beispielsweise die Information über den qualitativen oder quantitativen Gehalt an bestimmten körpereigenen oder körperfremden Stoffen, beispielsweise zum Blutgehalt von Glukose, Hormonen oder Elektrolyten, sowie der von Arzneistoffen oder Drogen.

Der Transfer von Stoffen in und durch die Haut folgt grundsätzlich den physikalischen Prinzipien einer passiven Diffusion, entsprechend den Ficks'schen Diffusionsgesetzen. Die Moleküle können dabei die Haut entweder transzellulär, d.h. durch die Zellen, oder interzellulär d.h. über die zwischen den Zellen befindlichen Interstitialräume, durchdringen. Sie können aber auch Wege über akzessorische Hautorgane, beispielsweise Haarfollikel und Schweissdrüsen, nehmen. Die oberste keratinhaltige Hautschicht, das Stratum comeum, stellt dabei eine wesentliche Barriere für die meisten Stoffe dar. Wenn eine Diffusion durch diese Schicht der Epidermis erreicht ist, permeieren die Moleküle leicht in die darunterliegende Dermis und werden dann von den Hautkapillaren absorbiert über die sie dann in den systemischen Kreislauf gelangen (Karzel, K. & Liedtke, R.K., Mechanismen transkutaner Resorption, Arzneim. Forsch/Drug Res. 11a (1989) 1487). Da die Diffusion ungerichtet nur den aktuellen Konzentrationgradienten folgt, gilt gleiches auch für den umgekehrten Passageweg, d.h. jenem aus den Kapillaren hin zur epidermalen Oberfläche, wo sich ebenfalls das Stratum Corneum als Hauptbarriere erweist.

Nach Kligman (Drug Dev. Industr. Pharm. 9:521-560, 1983) ist auch die Diffusion durch und in der Haut selbst primär ein temperaturabhängiger Prozess. Daraus ist zu erwarten, daß eine bestimmte Erhöhung der Hauttemperatur dort auch einen thermodynamischen Schub erhöht. Daraus ist wiederum zu erwarten, daß Wärmezufuhr dann, unter anderem, auch eine Freisetzung von Stoffen aus Depots, die in unter die Haut eingebracht sind, verstärkt. Hierzu fand sich beispielsweise bei Diabetikern nach Anwendung lokaler Wärme eine erhöhte Verschwinderate von vorher injiziertem 125J-markiertem Insulin aus dem subkutanen Gewebe, was auf eine diesbezüglich weitgehend lineare Erhöhung des kutanen Blutflusses zurückgeführt wurde (Hildebrandt. P. et al., J. Clin. Lab Invest (1985) 45(8) 685-90); sowie: Diabetes Res. 1987, 4(4) 179-81). Auch in einer anderen Studien an Gesunden (Sindelka, G., et al., Diabetologia (1994)37(4):377-80) war nach subkutaner Injektion die Höhe der Insulinspiegel im Serum statistisch signifikant korreliert mit der Hauttemperatur.

Dieser Effekt kann gleichzeitig durch mehrere Faktoren der physiologischen Regulation, allein oder durch deren Kombination, erzeugt sein. Beispielsweise durch Steigerungen in der zellulären Hautpermeabilität, durch Erhöhungen der lokalen Flüssigkeitszirkulationen, durch Steigerung der Permeabilität von Blutgefässwänden, zudem auch durch eine thermisch bedingte erhöhte chemische Löslichkeit der Stoffe. Schon Untersuchungen von Rowell et al. (J. Appl. Physiol. 28 (4) (1970) 415) zeigten, daß der kutane Blutfluss dabei um eine Rate von 3 L/min/°C Anstieg der Körperkerntemperatur verstärkt wird. Eine externe Erwärmung kann einen bis zu 12fachen Anstieg der Hautdurchblutung induzieren. Eine lokal begrenzte Erwärmung von Hautgeweben beeinflusst dabei aber nicht signifikant die Körper-Kerntemperatur, sie resultiert aber in einem lokalen Anstieg des subkutanen Blutflusses.

Verschiedene Studien wurden unternommen um zu zeigen, daß eine Verstärkung des kutanen Blutflusses auf Wärmeexposition auch die Pharmakokinetik transdermal verabreichter Stoffe ändert. Ergebnisse solcher Studien zeigen, daß eine externe Erwärmung die transdermale, als auch subkutane, Absorption verstärkt und dies dann in erhöhten Plasmakonzentrationen dieser Stoffe resultierte (Vanakoski, J. et al. Clin. Pharmacokinetics 34(4) (1998) 311-22).

Beispielsweise wurde die Beziehung zwischen kutanem Blutfluss und transdermaler Absorption von Nitroglycerin in einer Studie demonstriert, bei der Pflaster mit Nitroglycerin auf dem Oberarm plaziert wurden. Das Pflastergebiet wurde dabei isoliert mit einer Infrarot-Lampe erwärmt (Klemsdal et al., Eur. J. Clin. Pharmacol. 43 (1992) 625). Die Erwärmung verstärkte die lokale Blutperfusion und zur gleichen Zeit wurden die Nitroglycerin-Konzentrationen im Plasma um das zwei- bis dreifache gesteigert. Einem lokalen Abkühlen der Pflasterstelle mit Eis folgte wieder ein Abfall der Plasmakonzentrationen von Nitroglycerin, was zeigte, daß dieser Vorgang reversibel ist. In einer anderen Studie bestimmten Gupta et al. (J. Pain Symptom Management 7(3) (1992) Suppl: S17-S26) in-vitro den Effekt unterschiedlicher Temperaturen (zwischen 32°C and 37°C) auf den transdermalen Flux des analgetischen Stoffes Fentanyl. Über diesen Temperaturbereich verdoppelte sich in etwa die Flux-Rate. Basierend auf einem pharmakokinetischen Modell beruhte so ein Anstieg hauptsächlich auf zwei Faktoren, einer beschleunigten Freisetzung von Fentanyl aus dem technischen Reservoir des Pflasters sowie einer gesteigerten Hautpermeabilität.

Aus diesen Beispielen ergibt sich somit, daß bekannt ist, daß über eine Wärmeanwendung unter anderem auch transdermal pharmakodynamische Effekte auslösbar und verstärkbar sind, und eine Wärmeerzeugung mit verschiedenen physikalischen Mitteln oder auch mit chemischen Mitteln, beispielsweise durch Erzeugung chemisch exothermer Reaktionen, erfolgen kann.

Ein wesentlicher biologischer Mechanismus für das Phänomen scheinen dabei physiologisch wärmeinduzierte Erhöhungen des lokalen Hautblutflusses infolge lokaler Gefässerweiterungen sowie daraus auch resultierende lokale Änderungen der intradermalen Flüssigkeitszirkulation zu sein. Insgesamt umschliesst der Mechanismus damit sowohl eine Permeation durch Hautschichten, die Diffusion zwischen kutanem und subkutanem Gewebe, wie auch jene von Geweben in den systemischen Kreislauf. Die hierüber bewirkten Anstiege der Plasmakonzentrationen einiger Stoffe weisen somit darauf hin, daß eine technisch geeignete Vorrichtung zur Anwendung lokaler Wärme, für dazu geeignete Stoffe, beispielsweise unter anderem auch transdermal eine Freisetzung und Permeation steigern kann.

Im Gegensatz zu bereits einigen wenigen vorliegenden prinzipiellen Erkenntnissen, daß eine Anwendung lokaler Wärme auch einen dermal oder transdermalen therapeutischen Einsatz von Arzneistoffen fördern könne, ist zum Bereich dermaler oder transdermal diagnostischer Verfahren, d.h. zu dermal diagnostischen Verfahren oder Vorrichtungen, die ebenfalls auf einem lokal thermodynamischen Effekt beruhen oder durch diesen gefördert werden können, derzeit nichts bekannt.

Eine transdermal medizinisch-therapeutisch geeignete Auslösung biologischer Effekte, erfordert, daß die eingesetzten Stoffe, neben ihren geeigneten physikochemischen Eigenschaften wie beispielsweise Molukulargewicht und Löslichkeit, dazu auch in einer geeignet kontrollierten Form freigesetzt werden. Dies Ziel wird aber mit den bisher bekannten dermalen und transdermalen therapeutischen Systemen noch nicht erreicht. So stellen sowohl die derzeit eingesetzten transdermalen Pflastersysteme, wie auch die halbfesten pharmazeutischen Formulierungen, nur rein passive Diffusionssysteme dar. Der Transport der in ihnen enthaltenen Stoffe in den systemischen Kreislauf beruht somit nur auf der jeweiligen Konzentrationsdifferenz zwischen Wirkstoffreservoir in der pharmazeutischen Formulierung und Haut bzw. in der dazu nachfolgenden Phase deren Konzentrationsdifferenz zwischen subkutanem Gewebe und Blut. Dies ermöglicht solchen Vorrichtungen zwar eine kontinuierliche Stoff-Freigabe, aber es erlaubt keinerlei individuell erforderliche Änderungen und Anpassungen für eine reproduzierbar kontrollierte Permeation an eine individuell gegebene Situation. So wäre beispielsweise eine akute Dosiserhöhung eines Schmerzmittels bei einem Schmerzpatienten dann erforderlich, wenn das System kein genügende Dosis freisetzt um dessen akuten Schmerzzustand wirksam zu verringern.

Bekannt sind daher auch Forschungen um technische Vorrichtungen in solche therapeutischen Transdermalsysteme zu integrieren, die den Stofftransport durch die Haut verstärken oder die Freigabe der Stoffe aus dem Pflastersystem besser kontrollieren sollen. Dies beinhaltet den technischen Einsatz sowohl chemischer wie auch physikalischer Massnahmen.

Zu den chemischen Massnahmen gehören sogenannte chemische Enhancer. Dies sind Stoffe, die, über eine direkte chemische Beeinflussung der Hautstruktur, die Haut besser durchgängig machen sollen. Die Nachteile von solcherart Stoffen sind aber, daß sie die biologische Integrität der Haut chemisch zerstören und daraus erhebliche Hautirritationen und Nebenwirkungen erzeugen können. Weiterhin bekannt sind auch bestimmte chemische Agentien, sogenannte Rubefacientia, mit denen die Haut topisch gereizt wird und mit der diese dann neuronal reaktiv zu lokal vermehrter Durchblutung angeregt werden soll. Produkte mit Stoffen, die solche Wärmeempfindungen hervorrufen, sind teils als sogenannte topische "Rheumapflaster" bekannt. Die tatsächlichen regulatorischen Effekte solcher Reizstoffe sind jedoch umstritten und sie erzeugen oft nur über lokale Nervenreizung ein subjektives "Wärmegfühl", dies abgesehen davon, daß es sich aber auch hierbei nicht um einen kontrollierten Mechanismus für die Freisetzung handelt. Desweiteren sind auch chemische Massnahmen bekannt, bei denen über eine Auslösung von im Pflaster selbst enthaltenen Reagentien chemisch wärmerzeugende Reaktionen hervorgerufen werden, mit denen der Wirkstoff oder das Pflaster erwärmt wird. Diese Massnahme ist zwar prinzipiell geeignet auch eine Stoffpermeation zu erhöhen, sie erfolgt thermisch aber weitestgehend unkontrolliert und beinhaltet, durch den Einsatz der hierzu benötigten anorganischen Reagentien, zudem negative Sicherheits- und Verträglichkeitsaspekte für die Haut. So bezieht sich schon US Pat. 4,230,105 auf eine Bandage mit einem Arzneistoff und einer chemisch Wärme generierenden Vorrichtung. Auch US Pat. 4,898,592 beschreibt eine Vorrichtung für die Anwendung von erwärmten transdermal absorbierbaren Stoffen, wobei hier eine Lage mit einer transdermal absorbierbaren Substanz imprägniert ist und eine andere ein Wärmelement enthält. Die Ansprüche von US 4,685,911 beziehen sich ebenfalls auf die Wärmeanwendung mit einem mit chemischer Hilfe wärmegenerierenden Medium um die Absorption zu steigern. Ein Pflaster mit einer Vorrichtung zur direkten Erzeugung chemischer Wärme ist auch in US 6,306,431 beschrieben hier vorzugsweise mit einer Mischung aus Eisenpulver, aktiviertem Kohlenstoff, Salz und Wasser, bei dem dann, nach Entferung einer luftdichten Abdeckschicht, atmosphärischer Sauerstoff an das wärmegenerierende Gemisch gelangt, was hiernach die Auslösung einer exothermen Reaktion bewirkt. Dieser von US 6,306,431 für sich beanspruchte Mechanismus exothermer Wärmeerzeugung ist allerdings nicht neu, da auch schon US 4,685,911 exakt diese Form chemisch exothermer Wärmeerzeugung beschreibt. Im weiteren Rahmen ihrer Beschreibungen weisen einige dieser Techniken auch auf solcherart bekannte physikalische Allgemeinplätze hin, daß man statt Einsatz chemischer Energie beispielsweise auch elektrische Energie zur Wärmerzeugung einsetzen könne, wie auch, daß man elektrisch erzeugte Wärme auch mit Einsatz elektrischer Vorrichtungen kontrollieren könne.

Bekannte physikalische Massnahmen bei transdermalen Anwendungen eine bessere Kontrolle zu bewirken, sind auch solche mittels Elektrizität, beispielsweise durch Iontophorese, sowie mittels Einsatz von Vorrichtungen mit Ultraschall (bei dem u.a. ebenfalls indirekt subkutan Wärme entsteht) oder auch durch magnetische Vorrichtungen. Derzeit im therapeutischen Transdermalbereich technisch am weitesten entwickelt scheinen noch sogenannte iontophoretische Systeme. Bei diesem medizinisch-historisch schon lange bekannten, wie auch schon lange eingesetzten, technischen Prinzip, erfolgt eine Wanderung ionisierter Moleküle durch ein tangential in der Haut verlaufendes elektrisches Feld. Das elektrische Feld wird dabei mittels einer Stromquelle zwischen zwei auseinanderliegenden Elektroden des Pflasters erzeugt. Diese Systeme sind aber technisch sehr aufwendig, zudem relativ voluminös und sperrig, wie auch kostspielig. Sie ziehen zudem einige beträchtliche Probleme für die Hautverträglichkeit nach sich, was durch die direkte Einbeziehung der Haut als physisches Trägermedium für den erzeugten elektrischen Stromfluss bewirkt wird.

Aus dem vorher Aufgeführten ergibt sich, daß zwar über einzelne Aspekte zur therapeutischen Wärmeanwendung auf die Haut technisch isolierte Lösungsansätze verfolgt wurden. Eine Gesamtbetrachtung dermal thermodynamischer Vorgänge, d.h. solche, die den interaktiven Gesamtmechanismus und phyiologischen Auswirkungen topischer Wärmeeinwirkung in sich konsistent technisch beschreibt wie auch in entsprechend technisch integrierter und praktischer Form umsetzt, ist bisher nicht bekannt. All diesen technischen Vorrichtungen ist gemeinsam, daß sie ihre Effekte weitestgehend unkontrolliert auslösen, da sie nur einseitig gerichtet und irreversibel ablaufen, beispielsweise bei chemischen exothermen Reaktionen der Fall, die in ihrem weiteren Ablauf nicht mehr gesteuert werden können. Hierüber ist dann auch keine ausreichend bedarfsgerechte individuelle Dosierung möglich. Während sich im therapeutischen Bereich solcherart Vorrichtungen für dermale und trandermale Systeme mit einzelnen sowie miteinander nicht definiert abgestimmten technischen Prozesskomponenten aus Sicht der Informationstechnik nicht als kontrollierte Systeme einstufen lassen, finden sich im dermal und transdermal diagnostischen Bereich noch keinerlei Ansätze oder Vorrichtungen mit einem topisch thermodynamischem Ansatz.

Der Erfindung liegt die Aufgabe zu Grunde die Effizienz und Sicherheit topisch dermaler und transdermaler Therapien und Diagnosen zu verbessern.

Diese Aufgabe wird dadurch gelöst, daß pflasterartige Chip-Systeme zur thermodynamischen Kontrolle topisch dermaler und transdermaler Systeme eingesetzt werden, wobei diese als ein pflasterartig gestaltetes Mehrkomponentensystem so zusammengesetzt sind, daß sie aus einer in einer gemeinsamen Trägermatrix befindlichen elektrischen Energiequelle, einem als Thermocontroller dienenden programmierbaren Microprozessor und einer Aktivierungsschaltung bestehen, diese ihrerseits technisch mit einer Vorrichtung verbunden sind, die elektrisch induzierte Wärme erzeugt, und das pflasterartige Chip-System insgesamt an einem topisch dermalen oder transdermalen System komplementär so angebracht werden kann, daß die erzeugten Wärmeprofile derart in die topisch dermalen oder transdermalen Systeme übertragen wird, daß diese in kontrollierter Form thermodynamisch aktiviert werden.

In einer weiteren Ausbildung der Erfindung ist, um den praktischen Einsatz zu verbessern und auszuweiten, die gemeinsame Trägermatrix geometrisch in operative Funktionsektoren aufgeteilt ist, die untereinander elektrisch leitend miteinander verbunden sind, wobei die Verbindungen zwischen diesen Funktionssektoren reversibel ausgebildet sein kann.

In einer weiteren Ausbildung der Erfindung sind, um den praktischen Einsatz zu verbessern und auszuweiten die Matrices und technisch aktiven Bestandteile der pflasterartigen Chip-Systeme aus solchen Materialien zusammengesetzt, die mechanisch elastische oder plastische Eigenschaften besitzen, die optisch transparent oder opak sind, die elektrisch leitende oder magnetische Eigenschaften besitzen und die chemisch nicht-metallische natürliche oder synthetische Polymere oder metallische Materialien sind.

In einer weiteren Ausbildung der Erfindung sind, um den praktischen Einsatz zu verbessern und auszuweiten in die Vorrichtungen für spezifische Anwendungzwecke zusätzliche elektrische, elektronische, magnetische, mikromechanische, chemische oder chemotechnische Komponenten, oder Kombinationen hieraus, implementiert.

In einer weiteren Ausbildung der Erfindung erfolgt, um den praktischen Einsatz zu verbessern und auszuweiten, eine Kontrolle der induzierten Wärmeprofile entweder als open-loop Steuertechnik oder als durch Sensoren rückgekoppelte closed-loop Technik.

In einer weiteren Ausbildung der Erfindung sind, um den praktischen Einsatz zu verbessern und auszuweiten Einrichtungen zum Empfang und Aussenden von Fernsteuerungssignalen enthalten, wobei dies entweder physikalisch über Infarot, Ultraschall, elektromagnetische Wellen oder Lasertechniken oder chemosensorisch über chemisch volatile Stoffe erfolgen kann.

In einer weiteren Ausbildung der Erfindung kann, um den praktischen Einsatz zu verbessern und auszuweiten, der thermodynamische Aktor auch in Teilflächen, sowie diese mit unterschiedlichen Temperaturen, angesteuert werden.

In einer weiteren Ausbildung der Erfindung ist um den praktischen Einsatz zu verbessern und auszuweiten, der thermodynamische Aktor in allen zweidimensional möglichen Geometrien ausgestaltet.

In einer weiteren Ausbildung der Erfindung erfolgt, um den praktischen Einsatz zu verbessern und auszuweiten, die Produktion in Teilen oder im Ganzen technisch in Rolle-zu-Rolle Verfahren.

In einer weiteren Ausbildung der Erfindung werden, um den praktischen Einsatz zu verbessern und auszuweiten, die Vorrichtungen therapeutisch bei solchen dermalen und transdermalen Systemen eingesetzt, die keine pharmakologisch aktiven Wirkstoffe enthalten.

In einer weiteren Ausbildung der Erfindung werden, um den praktischen Einsatz zu verbessern und auszuweiten die Vorrichtungen therapeutisch im Rahmen einer regionalen Hyperthermie zur lokalen Erwärmung von Tumorzellen, eingesetzt, inbesondere solchen im Brustbereich, im Hautbereich oder im Genitalbereich.

In einer weiteren Ausbildung der Erfindung werden, um den praktischen Einsatz zu verbessern und auszuweiten diese therapeutisch bei topisch dermalen oder transdermalen Systemen eingesetzt, die als pharmakologisch aktive Wirkstoffe Nitroglycerin, Fentanyl, Sufentanil, Buprenorphin, Morphin, Hydromorphin, Lidocain, Indomethacin, Ibuprofen, Diclofenac, Piroxicam, Nikotin, Clonidin, Estradiol, Progesteron, Testosteron, Norethisteron, Oxybutinin, Buspiron, Scopolamin, enthalten, einschließlich ihrer chemischen Analoga, Derivate und Isomere sowie Salze als Einzelstoffe oder in Form von Kombinationen.

In einer weiteren Ausbildung der Erfindung sind, um den praktischen Einsatz zu verbessern und auszuweiten, wird diese therapeutisch bei dermalen oder transdermalen Systemen eingesetzt, die als pharmazeutische Formulierung halbfeste oder flüssige Formen wie insbesondere Salben, Gele, Cremes, Lotionen, Suspensionen oder Lösungen darstellen.

In einer weiteren Ausbildung der Erfindung wird, um den praktischen Einsatz zu verbessern und auszuweiten diese zur beschleunigten Desintegration epidermaler oder dermaler Wirkstoffdepots eingesetzt, insbesondere von Depots mit den Hormonen Insulin, Wachtumshormon, Estradiol, Progesteron, Testosteron, einschließlich ihrer chemischen Analoga.

In einer weiteren Ausbildung der Erfindung wird, um den praktischen Einsatz zu verbessern und auszuweiten, diese als pflasterartiges dermales Diagnosesystem zur Gewinnung und Analyse der natürlichen Flüssigkeit der Haut, Schweiss und dermale Interstitialflüssigkeit, eingesetzt, insbesondere zur Analyse der in dieser enthaltenen Substanzen Glukose, Laktat, Elektrolyte, Adrenalin, Kreatinin, Arzneimittel, Alkohol und Drogen.

In einer weiteren Ausbildung der Erfindung wird, um den praktischen Einsatz zu verbessern und auszuweiten diese als pflasterartiges nicht-invasives dermales oder transdermales Diagnosesystem eingesetzt, wobei Sammlung und Analyse der auf die Hautoberfläche austretenden Hautflüssigkeit mittels in diese integrierter Sammel- und Sensor-Vorrichtungen erfolgt, um die der thermodynamische Aktor zirkulär angeordnet ist, wobei die Hautflüssigkeit durch eine mit kapillaren Kanälen ausgestattete, plattenartige Sammelvorrichtung aufgenommen wird und die Flüssigkeit über mit dieser in Verbindung stehende elektronische Chemosensoren oder chemische Teststreifen analysiert und ausgewertet wird, und wobei dies zur nicht-invasiven Analyse von insbesondere Glukose, Laktat, Elektrolyten, Adrenalin, Kreatinin, Arzneimitteln, Alkohol und Drogen eingesetzt wird.

In einer weiteren Ausbildung der Erfindung wird, um den praktischen Einsatz zu verbessern und auszuweiten, diese als pflasterartiges dermales oder transdermales mikro-invasives Diagnosesystem eingesetzt, wobei die Sammlung und Analyse interstitieller Hautflüssigkeit mittels einer integrierten Sammel- und Sensor-Vorrichtung erfolgt um die der thermodynamische Aktor zirkulär angeordnet ist, wobei die interstitielle Hautflüssigkeit durch eine mit Mikrokanülen ausgestattete, plattenartige Sammelvorrichtung, die geeignet ist die oberste epidermale Hautlage zu durchdringen, aufgenommen oder kontaktiert wird und die Flüssigkeit über mit dieser in Verbindung stehende elektronische Chemosensoren oder chemische Teststreifen analysiert und ausgewertet wird, und wobei dies zur mikro-invasiven Analyse von insbesondere Glukose, Laktat, Elektrolyten, Adrenalin, Kreatinin, Arzneimitteln und Drogen eingesetzt wird.

In einer weiteren Ausbildung der Erfindung besteht, um den praktischen Einsatz zu verbessern und auszuweiten, die Sammel- und Leitvorrichtung für Hautflüssigkeit, insgesamt oder in Teilen, aus polymeren Hohlfasern, aus Mikrokanülen oder Hohlsonden aus metallischem, polymerem oder keramischem Material, wobei deren Anstellwinkel zur Perforation der Haut senkrecht, schräg oder tangential justiert sein kann, und wobei dieser Anstellwinkel auch durch weitere Vorrichtungen reversibel umjustiert werden kann.

In einer weiteren Ausbildung der Erfindung wird, um den praktischen Einsatz zu verbessern und auszuweiten, diese als pflasterartiges dermales oder transdermales nicht-invasives oder mikro-invasives Diagnose-Systeme eingesetzt, wobei die integrierten Sensor-Vorrichtungen als planare elektronische Chemosensoren ausgestaltet sind.

In einer weiteren Ausbildung der Erfindung wird, um den praktischen Einsatz zu verbessern und auszuweiten, diese als pflasterartiges dermales oder transdermales nicht-invasives oder mikro-invasives Diagnosesystem eingesetzt, bei dem die Sensor-Vorrichtung in dieses reversibel eingeschoben und entnommen werden kann.

In einer weiteren Ausbildung der Erfindung wird, um den praktischen Einsatz zu verbessern und auszuweiten, diese als pflasterartiges dermales oder transdermales nicht-invasives oder mikro-invasives Diagnosesystem eingesetzt, bei dem die Analyse der Flüssigkeit mittels eines in dieses reversibel einschiebbaren und entnehmbaren chemischen Teststreifen erfolgt.

In einer weiteren Ausbildung der Erfindung wird, um den praktischen Einsatz zu verbessern und auszuweiten, diese auch im Tierbereich eingesetzt.

Vorteile der Erfindung ergeben sich insbesondere dadurch, daß die beschriebenen pflasterartigen Chip-Systeme programmierte und "intelligent" kontrollierte Effekte ermöglichen und hierbei grundsätzlich in zweierlei Richtungen eingesetzt werden können. Zum einen, indem die von ihnen konduktiv emittierte Wärme direkt als biologischer Endeffekt genutzt wird, sowie zum anderen über einen Mechanismus den man als indirekten thermodynamischen Verstärker bezeichnen kann.

Ein direkter thermodynamischer Effekt im Bereich der Therapie, ist beispielsweise ein kontrollierter Relaxationseffekt über die Haut, beispielsweise bei Vorliegen neuromuskulär bedingter Spasmen oder neurologisch-metabolischer Erkrankungen. Hierbei handelt es sich somit um einen primären physikalischen therapeutischen Effekt, bei dem ein die Wirkung vermittelndes exogenes Pharmakon nicht nowendig ist. Eine weitere, ebenfalls direkte aber diagnostische, Anwendung ist beispielsweise die durch lokal kontrollierte Hyperthermie induzierte Aktivierung und Gewinnung von Hautflüssigkeiten mittels verstärkter Perspiration und intradermaler Hydratation, Schweiss und dermale Interstitial-Flüssigkeit, sowie deren unmittelbare Analyse mittels integrierter Mikrosensoren, beipielsweise über elektronische Chemosensoren, ionenselektive Sonden oder auch chemischen Teststreifen. Dies ermöglicht, je nach Lage, eine nicht-invasive oder mikro-invasive Diagnostik von körpereigenen Stoffen wie Elektrolyten und Glukose, aber auch der von pharmakologisch wirkenden Fremdstoffen, beispielsweise von Alkohol, Arzneistoffen, aber auch von Drogen. Insbesondere im Bereich der Blutglukose-Bestimmung bei Diabetikern ist eine nicht-invasive oder nur noch mikro-invasive Messung sehr vorteilhaft, da die derzeitige Vorgehensweise für diese Patienten schmerzhaft und umständlich ist, zudem zahlreiche Fehlerquellen beinhaltet. Da auch eine mikro-invasive Methode über dermale interstitielle Flüssigkeit als Analyt sich im Bereich der nozizeptorarmen sowie auch weitestgehend gefässfreien Epidermis abspielt, ist auch hier die Bestimmung schmerz- und blutfrei. Da die epidermale interstitielle Flüssigkeit zudem direkt mit den Blutwerten korreliert (Bantle JP, Thornas W, J. Lab. Clin. Med. 130 (1997) 436-41) ermöglicht sie auch mit sehr geringen Volumina eine vergleichbare Genauigkeit wie die bei Kapillarblut (Service FJ, O'Brien PC, et al: Diabetes Care, 20 (1997) 1426-1429).

Die zweite Anwendungsrichtung ist die indirekte Nutzung der Thermodynmik als sekundären Effekt, als Diffusions-Verstärker für dermale oder transdermale Freisetzungssysteme. Transdermale Freisetzungssysteme können dabei hier entweder feste mechanische Vorrichtungen sein, beispielsweise passive transdermale Pflastersysteme, oder halbfeste pharmazeutische Formulierungen, beispielsweise wirkstoffhaltige Hautsalben. Dermale Freisetzungssysteme sind auch unter der Hautoberfläche liegende Stoffdepots, beispielsweise retardierte Kristallsuspensionen oder kolloiddisperse Formulierungen oder Depotformulierungen aus biologisch kompatiblen Stoffen mit Schmerzmitteln oder mit Hormonen wie beispielsweise Insulin. Bei diesen kann dann thermodynamisch transkutan die Desintegration dieser, ansonsten nur sehr langsam löslichen, Depots gesteigert werden, woraus sich eine akut erhöhte Freisetzung der in ihnen enthaltenen Stoffe in den Blutkreislauf ergibt. Insgesamt kann daher durch diese sekundären Effekte aus unterschiedlichen dermalen oder transdermalen pharmazeutischen Formulierungen thermodynamisch zeitlich wie auch dosismässig kontrolliert, entweder auf akute Anforderung oder auch vorprogrammiert, sowohl eine stärkere Freisetzung als auch eine verstärkte Resorption erzeugt werden.

Als weitere Vorteile der Erfindung ergeben sich, daß die beschriebenen pflasterartigen Chip-Systeme die Wirkungen bisher rein passiver Systeme, nun auch individuell angepasst kontrollieren und steuern können. Durch die im System integrierten und interaktiven Steuer- und Kontroll-Komponenten erfolgt eine kontrollierte Beeinflussung der thermodynamischen Aktivitäten des angekoppelten Freigabesystems. Dies kann über länger vorgegebene Zeiträume reproduzierbar und in vorgegebenen Dosen als auch auf direkte Anforderung des Benutzers erfolgen.

Das physikalische Grundsystem der pflasterartigen Chip-Systeme lässt sich für diese komplexe Anwenung zudem mathematisch insgesamt als indirekte partielle Aktivierung der Bateman-Funktion - der sich als pharmakokinetische Resultante aus Invasions- und Evasions-Prozessen ergebenden Zeit-Konzentrationskurve von Stoffen im Blut, somit als eine Folge der Aktivierung der Diffusions-Bedingungen, beschreiben. Diese Aktivierung erfolgt dabei über den chipkontrollierten konduktiven Wärmetransfer, der sich aus dem Fourier'schen Gesetz der Wärmekonduktion ableitet, durch welche somit quasi als "Fourier-Systeme" arbeitende pflasterartigen Chip-Systeme eine kontrollierte "emissive Power" bewirken.

Die transdermale Diffusion steigt dabei proportional mit dem Anstieg der lokalen Temperatur, entsprechend Fick'schem Gesetz $dQ/dt = DF(C_1-C_2/d)$. Die konduktive Verstärkung folgt Fourier's Gesetz des konduktiven Wärmetransfers $dQ/dt = -\lambda A (dT/dx)$. Dieses bestimmt den Wärmeflussvektor Q für das gegebene Temperaturprofil T und die Proportionalitätsrate des eingesetzten Materials (thermische Konduktivität $\lambda$). Die Rate des Wärmeflusses $dQ/dt$ durch einen homogenen Feststoff ist proportional zur Fläche A, d.h. dem Ausschnitt der im rechten Winkel zur Richtung des Wärmeflusses steht und zur Temperaturdifferenz entlang dem Pfad des Wärmeflusses $dT/dx$. Dies wiederum führt, über Verstärkung der Diffusionsparameter, zu Änderungen in den Absorptions-Parametern der Bateman Funktion: $C = C_o \cdot k_a/k_a - k_e (e^{-ke \cdot t} - e^{-ka \cdot t})$, wobei hier: $C_o$ = fiktive initiale Konzentration, $k_a$ = Konstante der Invasionsgeschwindigkeit, $k_e$ = Konstante der Eliminationsgeschwindigkeit. Die globale Zusammenfassung der vorgenannten Interdependenzen ist daher:

$$dQ/dt = -\lambda\ A\ dT\ /dx \Rightarrow dQ/dt = D\ F\ (C_1-C_2/d) \Rightarrow C = C_o \cdot k_a/k_a - k_e\ (e^{-ke \cdot t} - e^{-ka \cdot t})$$

Damit eröffnet sich, als ein weiterer Vorteil, die Möglichkeit, die thermodynamisch zu erwartenden biologischen Effekte rechnerisch abzuschätzen.

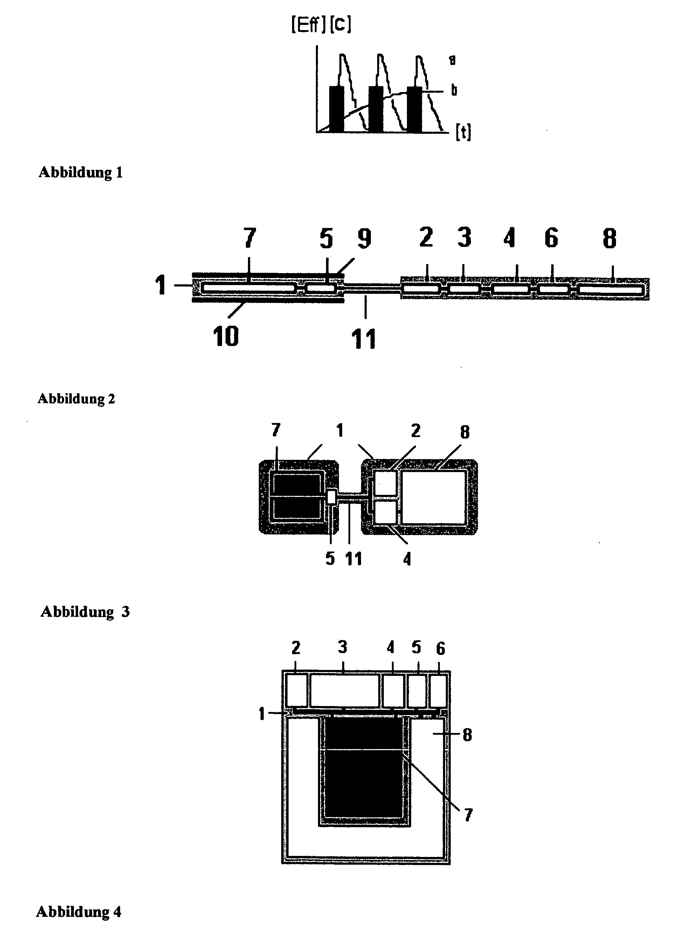

In **Abbildung 1** ist diese Basischarakteristik schematisch dargestellt. Bei Auslösung zeitlich begrenzter definiert induzierter Wärmepulse (graue Säulen) kommt es bei einem hierüber thermodynamisch aktivierten passiven Transdermalsystem (Kurve a) reproduzierbar zu pulsartigen Erhöhungen der Stoff-Freisetzung und hieraus zu einer erhöhten Serumkonzentration des Stoffes [C], bzw. damit der hierzu korrelierenden Effektausprägung [Eff], über die Zeit [t]. Nach Ende der thermodynamischen Aktivierung fällt die Serumkurve, bzw. damit die Ausprägung des Effektes dann wieder ab. Demgegenüber setzt ein rein diffusionsabängiges, passives System (Kurve b) ohne thermodynamische Aktivierung die in ihm enthaltenen Wirkstoffe nur entsprechend den bestehenden Konzentrationsdifferenzen frei, folgt somit nur den Charakteristiken einer kontinuierlichen Invasionskinetik 1. Ordnung.

Weitere Vorteile der Erfindung sind, daß je nach Erfordernis und Anwendungsziel die Kontrollelemente der Chip-Systeme entweder als Bedarfs-Systeme, beispielsweise in einem festprogrammierten Modus mit Wahlmöglichkeiten (open-loop), oder als über einzelne oder mehrere integrierte Sensoren rückkoppelnde Systeme (closed-loop) mit verschiedenen Programmoptionen ausgestaltet sein können. In Abhängigkeit des Umfanges der jeweiligen Progammierung des Mikroprozessors lassen sich dazu unterschiedliche Anwendungen ermöglichen. So können über frei oder fest vorprogammierte Zeit-Wärme-Profile beispielsweise die konsekutiven Freisetzungskinetiken sowohl physiologischen wie auch individuellen Bedingungen und Erfordernissen angepasst werden. Permeationen lassen sich dann beispielsweise in entweder bestimmten Zeitintervallen oder zu solchen Tageszeiten pulsen, daß sie besser den Gegebenheiten sogenannter zirkadianer Rhythmen folgen. Hierüber können auch adaptive Gegenreaktionen verringert werden, beispielsweise bei Stoffen die Toleranzphänomene aufweisen wie Nitrogylcerin. Wie es Abbildung 1 schematisch zeigt ist damit auch eine bedarfsweise akute individuelle Dosisanpassung möglich, z.B. bei einer klinisch rascher erforderlichen höheren Dosis eines Schmerzmittels, was mit passiven Systemen nicht möglich ist Durch Implementierung von Fernsteuerungselementen (Remote Control) in das pflasterartige Chip-System, beispielsweise über Infrarot, kann eine Dosisänderung bei einem Patienten ggfs. auch durch den behandenden Arzt oder das Pflegepersonal elektronisch veranlasst werden. In Erweiterung dieser operativen Optionen können die entsprechenden Behandlungsmassnahmen auch im Mikroprozessor gespeichert und drahtlos über Computer-Schnittstellen ausgelesen und in einem Computer weiterverarbeitet und dokumentiert werden.

Diese individualisierten Anpassungen der Arzneidosis erhöhen dabei sowohl die Lebensqualität der Patienten wie auch die Therapie-Sicherheit durch Verringerung unerwünschter Arzneiwirkungen. Geeignete Anwendungen sind beispielsweise Schmerz-Therapien oder Therapien im Bereich zentraler Stimmungsstörungen. Im Gegensatz zu den iontophoretisch arbeitenden Transdermalsystemen kommt die Haut bei einem thermodynamisch aktivierten System weder direkt mit elektrischen Teilen noch mit Stromflüssen in Berührung. Die Anwendungssicherheit wie auch die lokale Hautverträglichkeit ist daher deutlich höher als die iontophoretischer Systeme. Zudem ist die Anwendungbreite höher, da iontophoretische Systeme nur mit ionisierbaren Stoffen arbeiten können. Die erforderlichen lokalen Temperaturdifferenzen zur thermodynamischen Aktivierung der angekoppelten Systeme betragen nur wenige Grad Celsius, sind daher sowohl lokal für die Haut wie auch den Gesamtorganismus unbedenklich.

Da die pflasterartigen Chip-Systeme als integrierter und interaktiv kontrollierter thermodynamischer Aktivator arbeiten ist ein Hauptgebiet - im Bereich therapeutischer transdermaler Anwendungen - auch ihre Kopplung mit bereits bestehenden und klinisch angewendeten passiven Transdermaltherapien. Sie können somit auch bereits schon bestehende Therapien optimieren indem sie diesen durch Ankopplung eine bessere Steuerbarkeit und individuelle Kontrolle eröffnen. Die Auslösung solcher nun transdermal "intelligent" kontrollierten Reaktionen zeigt daher gegenüber den Effekten rein passiver Transdermalsysteme signifikante medizinische Vorteile.

Ein technischer Vorteil ist, daß die pflasterartigen Chip-Systeme mit bereits üblichen Produktionsmitteln in großen Anzahlen wirtschaftlich, exakt genormt sowie reproduzierbar gefertigt sowie auch anwendungsbezogen variabel dimensioniert werden können. Durch ihre neuartige insgesamt flexibel pflasterartige Ausbildung, die Einlagerung in flexible Materialien sowie die Ausprägung mechanisch flexibler Komponenten erlauben die pflasterartigen Chip-Systeme auch Rolle-zu-Rolle Produktionsformen, so wie sie beispielsweise auch bei Drucktechniken oder in Teilprozessen der Mikroelektronik eingesetzt werden. Dies ist mit sonst üblichen festen Techniken und deren starren Trägerkomponenten nicht möglich. Zudem ermöglicht und vereinfacht es auch ihre Implementierung in bereits bestehende pharmazeutische Rollezu-Rolle-Produktionen wie sie beispielsweise bei Transdermalpflastern und bei Bandagen eingesetzt werden. Dabei lassen sich die pflasterartigen Chip-Systeme zudem dimensionsmässig komplementär an bestehende dermale oder transdermale Systeme anpassen und an diese fixieren. Desweiteren können sie auch im Rollezu-Rolle Verfahren automatisiert auf ihre Funktionsfähigkeit getestet werden.

Prinzipielle technische Beispiele der Erfindung sind, ohne es dabei aber auf diese Beispiele technisch beschränken zu wollen, nachfolgend erläutert:

**Abbildung 2** zeigt im schematischen Querschnitt den prinzipiellen Aufbau eines pflasterartigen Chip-Systems, bei dem funktionell verschiedene Teile der gemeinsamem flexiblen Trägermatrix flächenmässig in getrennten Sektoren ausgestaltet sind. Dabei befindet sich in einer gemeinsamen Trägermatrix (1) aus flexiblem Polymer ein extern zugänglicher Schalter (2) zur Aktivierung des Systems, optional ein Display (3), beispielsweise aus lichtemittierenden Dioden, ein mit verschiedenen Anschluss-Optionen ausgestatteter progammierbarer Mikroprozessor (4) als zentraler Kontroller, optional auch ein spezifischer operationeller Sensor-Device (5) für einen oder mehrere Sensoren, beispielsweise zur Temperatur- oder Feuchtigkeits -Kontrolle oder zur Ermittlung spezifischer Stoffkonzentrationen, optional eine Sende- und Empfangsstation für drahtlose Fernbedienung (6), beispielsweise eine Schnittstelle für Infrarot-Ansteuerung. Diskrete Teile wie Kondensatoren und Widerstände sind in diesem Schema nicht aufgeführt. Die Bestandteile 2-6 sind dabei direkt und interaktiv mit einer elektrisch Wärme erzeugenden Vorrichtung (7), dem thermodynamischen Aktor, verbunden. Dies kann beispielsweise eine flexible gedruckte Widerstandsschaltung sein oder auch eine kontinuierliche dünne Karbonschicht, die auf flexible Folie aufgebracht sind. Der Aufbau ist zudem mit einer Energiequelle (8) verbunden, die beispielsweise aus einer mechanisch flexibel ausgestalteten, ultraflachen Lithium-Polymer Batterie besteht.

Dabei ist der Mikroprozessor hier, zwecks Dimensionseinsparung in der Höhe, ohne isolierende Schutzhülle eingebracht, somit als "nackte" Prozessorstruktur in die flexible Matrix gebettet, und kann zudem noch durch zusätzliche Bearbeitungsverfahren weiter schichtverdünnend mechanisch elastifiziert sein. Das Batterievolumen ist durch ein spezifisches Design flachflächig verteilt. Auf der Oberseite der Trägermatrix liegt, in diesem Beispiel, noch eine dünne Abdeckschicht mit wärmereflektierender Kaschierung (9), die einseitig eine nach oben gerichtete Wärmeabstrahlung verringert. Auf der Unterseite der Matrix befindet sich eine thermoresistente Adhäsivschicht (10). Diese dient der reversiblen Fixierung des pflasterartigen Chip-Systems auf einer mechanischen Oberfläche, beispielsweise auf einem transdermalen Pflaster-System mit dem das Chip-System gekoppelt wird, oder auch einer reversiblen Fixierung auf der Haut, beispielsweise bei Anwendung für ein dermales System. Der Matrixteil mit dem thermodynamischem Aktor und das Kontroll-Panel sind durch eine dünne Matrix-Brücke (11) getrennt ausgebildet. Diese Art von "Tender-Vorrichtung" erhöht die Möglichkeit flexibler Anbringungen, beispielsweise solcher, die mit Torsionen verbunden sind. Die Gesamtdicke solcherart flexibler pflasterartiger Chip-Systeme liegt meist deutlich unter 1 mm und die Gesamthöhe solcher Vorrichtungen kann zwischen 10 µm und 2000 µm liegen. Der Aktor ist in der Lage, beispielsweise bei Aufbringung auf Haut, in vorgewählten Zeitintervallen in dem dort unter ihm liegenden Hautoberfächenareal kontrolliert regionale Temperatursteigerung um wahlweise zwischen 1°Celsisus bis 6°C zu erbringen, dies entsprechend absoluten Temperaturbereichen zwischen ca. 36° bis 42°C. Längere Temperaturen über 42°C sind in der Regel für die Haut schädlich.

**Abbildung 3** zeigt die Vorrichtung der Abbildung 2 in einer schematischen Aufsicht, wobei hier aber Implementierung eines Display und einer Remote Control weggelassen sind. Wie in Abbildung 2 ist der thermodynamische Aktor (7) über einen operationellen Sensor (5) elektrisch leitend und flexibel über einen Steg (11) aus Material der Trägermatrix mit einem weiteren Teil, der Schalter (2), Mikroprozessor (4) und Energieversorgung (8) trägt, verbunden, wobei diese Verbindung (11) auch optional mittels einer reversiblen elektrischen Ankopplung, beispielsweise mittels einer dort integrierten Stecker- oder Magnet-Kupplung, erfolgen kann. Eine weitere Segmentierung der Kontroll-Panel Matrix ist technisch möglich. Durch diese Form der Ausgestaltung ergibt sich weitere Erhöhung der räumlichen Flexibilität und Variabilität, so beispielsweise bei Oberflächen unterschiedlicher Topografie, wie auch eine Austauschbarkeit von Sensoren und/oder Energiequellen bei unterschiedlichen Erfordernissen.

**Abbildung 4** zeigt gegenüber den Abbildungen 2 und 3 schematisch in der Aufsicht ein voll integriertes System, bei dem alle Komponenten in der flexiblen Matrix (1) d.h. Aktor (7) und gesamtes Kontroll-Panel mit seinen verschiedenen Steuerungs- und Kontroll-Komponenten (2-6) direkt räumlich zusammenhängend angeordnet sind. Der thermodynamische Aktor (7) ist in diesem Design technisch zentriert in der Matrix angebracht. Die als Energiequelle dienende Batterie (8) ist zwecks einerseits Reduktion ihrer Höhendimension sowie andererseits Erhöhung ihrer Flexibilität flächig und U-förmig um den gesamten Aktor ausgebreitet.

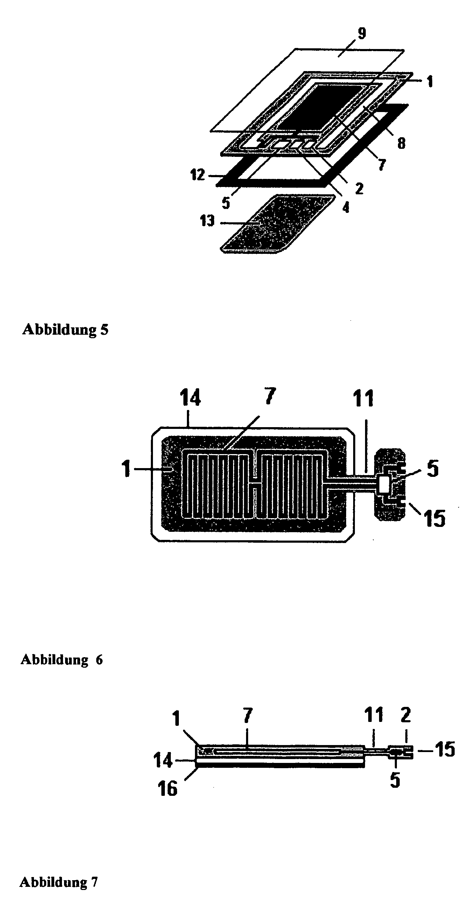

**Abbildung 5** zeigt als Explosionsschema ebenfalls ein voll integriertes System für einen therapeutischen Anwendungszweck, bei dem alle Komponenten direkt räumlich zusammenhängend angeordnet sind. Bei diesem pflasterartigen Chip-System ist die Klebeschicht des Chip-Systems (12) zirkulär ausgestaltet, was im Kontext mit einem anzukoppelnden passiven Transdermalsystem (13) steht. Dieses System wird somit um das Areal eines bereits auf der Haut befindlichen Transdermalsystems zirkulär herum aufgeklebt. Solche Konfiguration hat den Vorteil, daß wenn das therapeutische Transdermalsystem aus einer halbfesten pharmazeutischen Formulierung besteht, bespielsweise einem wirkstoffhaltigen Gel, das System auf solch einer halbfesten Schicht mechanisch nicht fixiert werden könnte. Somit besteht in diesem Falle zwischen dem pflasterartigen Chip-System und dem Transdermalsystem auch keine direkte mechanische Verbindung. Das gleiche technische Design kann aber gegebenenfalls bei einem festen Pflaster-System eingesetzt werden, wenn dort ebenfalls keine mechanische Verbindung erfolgen soll. Mit der Auslösung des im Mikroprozessor (4) enthaltenen Programms über den Schalter (2) wird dann über den Aktor (7) die im angekoppelten Transdermalsystem (13) befindliche Wirkstoffmatrix konduktiv thermodynamisch aktiviert. Hierauf kommt es in dem angekoppelten Transdermalsystem zu einer gegenüber der bisher rein passiven Diffusionsrate um einen vorprogrammiert thermodynamischen Aktivitätsfaktor gesteigerten Wirkstoff-Freisetzung.

**Abbildung 6** zeigt in schematischer Aufsicht das Grunddesign eines pflasterartigen Chip-Systems zur physikalischen direkten therapeutischen Anwendung von lokaler Hyperthermie, beispielsweise bei neuro-muskulär bedingten Schmerzen. Hierbei ist die Matrix (1) mit dem in ihr enthaltenen Aktor (7) direkt auf ein medizinisches Pflaster (14) klebend aufgebracht. Der Aktor ist hier über eine Matrixbrücke (11) mit einem weiteren flexiblen Matrixteil elektrisch leitend verbunden. Dieser zweite Teil enthält den Mikroprozessor (5), der seinerseits leitend mit einer Steckverbindung (15) verbunden ist. Über diese Steckverbindung kann über eine flexible Leitung dem System beispielsweise elektrische Energie, zugeführt werden.

**Abbildung 7** zeigt den gleichen Aufbau wie Abbildung 6 im schematischen Querschnitt. Hierbei ist noch die unter dem medizinische Pflaster (14) befindliche Hautklebeschicht (16) aufgeführt.

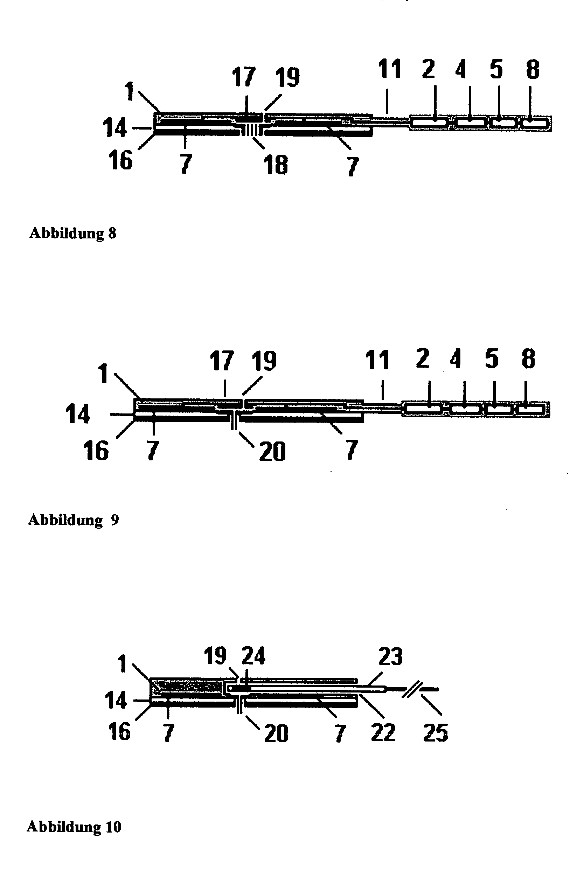

**Abbildung 8** zeigt im schematischen Querschnitt eine Modifikation des Grundaufbaus für ein dermal diagnostisches System. In diesem technischen Beispiel handelt es sich um Sammlung von auf der Hautoberfläche austretender Hautflüssigkeit bei thermodynamisch induzierter Hydratation, sowie deren quantitative oder qualitative Analyse mittels integrierter Mikrosensor-Vorrichtung, beispielsweise einem elektronischen Chemosensor oder auch einem chemischen Teststreifen. Der in der flexiblen Trägermatrix (1) liegende thermodynamische Aktor (7) ist hier zirkulär um eine in die Matrix integrierte planare Sensor-Vorrichtung (17) ausgelegt. Die auf die Hautoberfläche ausgetretene Flüssigkeit wird durch eine plattenartige Vorrichtung mit einem oder mehrere kapillaren Kanälen (18) kohäsiv aufgenommen und auf ihre, dem Sensor-Messareal direkt gegenüberliegende, Oberfläche geleitet. Die Oberfläche der Vorrichtung bildet dabei topografisch den Bestandteil einer integrierten Mikrokammer, die zudem mit einem oder mehreren Entlüftungskanälen (19) verbunden ist. Die Sensor-Vorrichtung ihrerseits ist mit einem spezifischen operationellen Sensorprozessor (5) und dem Mikroprozessor (4) verbunden. Das System ist noch auf ein medizinisches Trägerpflaster (14) aufgebracht, das an der Unterseite mit einer Hautklebeschicht (16) versehen ist und im Areal der Kapillaren-Vorrichtung (18) eine Perforation zur Haut enthält. Anwendungen sind beispielsweise schmerzlose und blutfreie nicht-invasive Pflastersysteme zur Analyse solcher Stoffe, die aus der systemischen Zirkulation über die Hautanhangsorgane und/oder interstitiell oder transzellulär auf die Hautoberfäche gelangen und technisch mit Hilfe von Chemosensoren, Mikrosonden oder Teststreifen nachweisbar sind, beispielsweise Elektrolyte, Adrenalin, Glukose, Laktat, bestimmte Arzneimittel, Alkohol oder bestimmte Drogen. Befundauswertung kann über eine PC-Schnittstelle des Mikroprozessors erfolgen, die zuerst im PC ausgelesen und dann dort verarbeitet und dokumentiert werden kann.

**Abbildung 9** zeigt im schematischen Querschnitt eine weitere Modifikation des Grundaufbaus des pflasterartigen Chip-Systems für einen dermalen diagnostischen Zweck. Hier handelt es sich um eine mikro-invasive quantitative oder qualitative Analyse interstitieller Flüssigkeit (ISF) der epidermalen Hautschicht, mittels eines in die Matrix (1) integrierten spezifischen planaren Mikrosensors (17). Die Hydratation dieser Hautschicht wird, zusätzlich zu der bereits verstärkten Hydratation durch die Pflasterbedingte mechanische Okklusion der Hautoberfläche, thermodynamisch durch den Aktor (7) verstärkt. Dieser ist zirkulär um den Sensor angebracht ist. Die ISF der Epidermis wird mittels einer plattenartige Vorrichtung mit einer oder mehreren Mikrokanülen (20), die ihrerseits in die ISF der epidermalen Hautschicht eintauchen, aufgenommen, teils kohäsiv aufgesaugt, und auf ihre dem Sensor-Messareal gegenüberliegende Oberfläche geleitet. Ein wesentlicher physikalische Mechanismus hierbei ist aber die temperaturbedingte Steigerung des subkutanen Blutflusses mit konsekutiv vermehrter Hydratation des epidermalen zellulären und interzellulären Verteilungsraumes. Da diese induzierte regionale Hyperthermie unter anderem auch die interstitielle Zirkulation der epidermalen ISF erhöht, entsteht eine Art Umlaufpumpen-Mechanismus, so daß die Aufnahme und Weiterleitung der epidermalen Flüssigkeit über einen regional erhöhten hydrostatischen Druck, im Sinne eines Artesischen Brunnenprinzipes, gefördert wird. Die Oberfläche der Aufnahmevorrichtung bildet topografisch den Bestandteil einer integrierten Mikrokammer, die zudem mit einem oder mehreren Entlüftungskanälen (19) verbunden ist. Wie zum Abbildungsbeispiel 8 beschrieben ist auch diese Modifikation noch auf ein medizinisches Trägerpflaster (14) aufgebracht, das an der Unterseite mit einer Hautklebeschicht (16) versehen und das Pflasterareal um die epidermalen Mikrokanülen (20) zur Haut hin perforiert.

Im Falle einer spezifischen Glukose-Bestimmung kann die chemische Reaktion beispielsweise mit Hilfe von in den Sensor integriertem Enzym Glukose-Oxidase erfolgen und hierbei das Reaktionspotential dann beispielsweise amperometrisch dargestellt werden.

Bei eine Modifikation des gleichen Systems mit Einsatz eines nichtplanaren Sensors, bei dem das Enzym Glukos-Oxidase bereits auf die Kanülen dotiert ist, kann die Flüssigkeit auch schon epidermal in-situ analysiert werden, was dann auch Möglichkeit kontinuierlicher in-situ Messung eröffent. Da die induzierte regionale Hyperthermie auch die interstitielle Zirkulation der epidermalen ISF erhöht, kann dort, im Sinne einer Art Umlaufpumpen-Mechanismus, permanent ein Gradient für den Sensor aufrecht erhalten werden.

Die sehr dünnen oberen epidermalen Schichten sind weitestgehend frei von Blutgefässen und terminalen Schmerzrezeptoren, erfordern daher für die Mikrokanülen nur invasive Distanzen von ca. 1-1,5 mm, wobei nur die oberste sehr dünne Keratin-Lage der Haut, das Stratum Corneum durchdrungen werden muss. Für dieses technische Beispiel sind geeignete Anwendungen daher schmerzlose und blutfreie mikro-invasiv dermale diagnostische Pflastersysteme zur Analyse solcher Stoffe, die aus der systemischen Zirkulation in die interstitielle Flüssigkeit der epidermalen Hautschichten gelangen, und technisch, beispielsweise mit elektronischen Chemosensoren, oder auch chemischen Teststreifen, nachweisbar sind. Dazu gehören Glukose, Laktat, Elektrolyte, Adrenalin, Kreatinin, bestimmte Arzneimittel oder auch bestimmte Drogen. Die Analyse und Auswertung erfolgt wie zu Abbildungsbeispiel 8 beschrieben. Eine Befundauswertung und Befunddokumentation kann über eine PC-Schnittstelle des Mikroprozessors erfolgen.

Abbildung 10 zeigt im schematischen Querschnitt eine weitere Modifikation des Grundaufbaus des pflasterartigen Chip-Systems für einen dermalen diagnostischen Zweck. Hier handelt es sich um die mikro-invasive Variante der Analyse interstitieller Flüssigkeit (ISF) der epidermalen Hautschicht, wie sie schon in Abbildung 9 dargestellt ist. In dieser Modifikation befidet sich aber in der Trägermatrix des Systems eine vorgefertigte schlitzartige Führungseinrichtung (22), über die entweder eine planar ausgebildete elektronische Sensor-Vorrichtung oder ein üblicher chemischer Teststreifen (23) reversibel in das System eingeführt werden kann. Im eingeschobenen Zustand sind das Lesefenster des Sensors, bzw. das Reaktionsareal des Testreifens (24) direkt über der ausgetretenen ISF positioniert und mit dieser in Kontakt. Im Falle eines elektronischen Sensors ist dieser über eine gemeinsame Mess- und Versorgungsgleitung (25), dann mit dem auswertenden Kontroll-Panel elektrisch verbunden. Diese Modifikation erlaubt daher, bei gleichem Grundprinzip, den wiederholten Einsatz des gleichen Sensors bei unterschiedlichen Pflaster.Systemen. Im Falle eines chemischen Teststreifens enthält das Pflaster noch einen elektrischen Kontakt zum Anschluss der elektischen Versorgung des thermodynamischen Aktors.

Eine gleichartige planare Ausbildung mit reversiblem Sensor- oder Teststreifen-Einsatz, kann natürlich auch für das nicht-invasive System, ensprechend Abbildung 8, für die Analyse von Flüssigkeiten der Hautoberfläche eingesetzt werden.

Die Erfindung betrifft pflasterartige Chip-Systeme zur thermodynamischen Kontrolle topisch dermaler und transdermaler Systeme, insbesondere zur Verbesserung von Effizienz und Sicherheit dermaler und transdermaler Therapien und Diagnosen. Informationstechnisch repräsentieren die Systeme komplexe technische Vorrichtungen, die gegenüber üblichen passiven Systemen durch progammierbare wie auch individuelle Steuerung kontrollierte und intelligente Systeme darstellen. Ihre Implementierung in passive dermale oder transdermale therapeutische Systeme erfordert keine technischen Eingriffe in den bestehenden Aufbau solcher Systeme. Im Bereich nicht-invasiver und mikro-invasiver dermaler und transdermaler Diagnostik eröffnen die pflasterartigen Chip-Systeme zudem neue Anwendungsmöglichkeiten.

**Patentansprüche**

1. Flexible Chip-Systeme zur thermodynamischen Aktivierung und Kontrolle dermaler und transdermaler Systeme, **dadurch gekennzeichnet, daß** pflasterartige Chip-Systeme zur thermodynamischen Kontrolle topisch dermaler und transdermaler Systeme eingesetzt werden, wobei diese als ein pflasterartig gestaltetes Mehrkomponentensystem so zusammengesetzt sind, daß sie aus einer in einer gemeinsamen Trägermatrix befindlichen elektrischen Energiequelle, einem als Thermocontroller dienenden programmierbaren Microprozessor und einer Aktivierungsschaltung bestehen, diese ihrerseits technisch direkt mit einer Vorrichtung verbunden sind die elektrisch induzierte Wärme erzeugt, und das pflasterartige Chipsystem insgesamt an einem topisch dermalen oder transdermalen System komplementär so angebracht werden kann, daß die erzeugten Wärmeprofile derart in die topisch dermalen oder transdermalen Systeme übertragen werden, daß diese in kontrollierter Form thermodynamisch aktiviert werden.

2. Vorrichtungen nach Anspruch 1, **dadurch kennzeichnet, daß** die gemeinsame Trägermatrix geometrisch in operative Funktionssektoren aufgeteilt ist, die untereinander elektrisch leitend miteinander verbunden sind, wobei die Verbindungen zwischen diesen Funktionsektoren reversibel ausgebildet sein kann.

3. Vorrichtungen nach vorhergehenden Ansprüchen, **dadurch gekennzeichnet, daß** die Matrices und technisch aktiven Bestandteile der pflasterartigen Chip-Systeme aus solchen Materialien zusammengesetzt sind, die mechanisch elastische oder plastische Eigenschaften besitzen, die optisch transparent oder opak sind, die elektrisch leitende oder magnetische Eigenschaften besitzen und die chemisch nicht-metallische natürliche oder synthetische Polymere oder metallische Materialien sind.

4. Vorrichtungen nach vorhergehenden Ansprüchen, **dadurch kennzeichnet, daß** in diese für spezifische Anwendungzwecke zusätzliche elektrische, elektronische, magnetische, mikromechanische, chemische oder chemotechnische Komponenten, oder Kombinationen hieraus, implementiert sind.

5. Vorrichtungen nach vorhergehenden Ansprüchen, **dadurch kennzeichnet, daß** eine Kontrolle der induzierten Wärmeprofile entweder als open-loop Steuertechnik oder als durch Sensoren rückgekoppelte closed-loop Technik erfolgt.

6. Vorrichtungen nach vorhergehenden Ansprüchen, **dadurch gekennzeichnet, daß** diese über Einrichtungen zum Empfang und Aussenden von Fernsteuerungssignalen enthalten, wobei dies entweder physikalisch über Infarot, Ultraschall, elektromagnetische Wellen oder Lasertechniken oder chemosensorisch über chemisch volatile Stoffe erfolgen kann.

7. Vorrichtungen nach vorhergehenden Ansprüchen, **dadurch gekennzeichnet, daß** der thermodynamische Aktor auch in Teilflächen, sowie diese auch mit unterschiedlichen Temperaturen, angesteuert werden können.

8. Vorrichtungen nach vorhergehenden Ansprüchen, daß der thermodynamische Aktor in allen zweidimensional möglichen Geometrien ausgestaltet sein kann.

9. Vorrichtungen nach vorhergehenden Ansprüchen, daß deren Produktion in Teilen oder im Ganzen technisch in Rolle-zu-Rolle Verfahren erfolgt.

10. Vorrichtungen nach vorhergehenden Ansprüchen, **dadurch gekennzeichnet, daß** diese therapeutisch bei solchen dermalen und transdermalen Systemen eingesetzt werden, die keine pharmakologisch aktiven Wirkstoffe enthalten.

11. Vorrichtungen nach vorhergehenden Ansprüchen, **dadurch gekennzeichnet, daß** diese therapeutisch im Rahmen einer regionalen Hyperthermie zur lokalen Erwärmung von Tumorzellen, eingesetzt werden, inbesondere solchen bei Tumoren im Brustbereich, im Hautbereich oder im Genitalbereich.

12. Vorrichtungen nach vorhergehenden Ansprüchen, **dadurch gekennzeichnet, daß** diese therapeutisch bei topisch dermalen oder transdermalen Systemen eingesetzt werden, die als pharmakologisch aktive Wirkstoffe Nitroglycerin, Fentanyl, Sufentanil, Buprenorphin, Morphin, Hydromorphin, Lidocain, Indomethacin, Ibuprofen, Diclofenac, Piroxicam, Nikotin, Clonidin, Estradiol, Progesteron, Testosteron, Norethisteron, Oxybutinin, Buspiron, Scopolamin, enthalten, einschließlich ihrer chemischen Analoga, Derivate und Isomere sowie Salze als Einzelstoffe oder in Form von Kombinationen.

13. Vorrichtungen nach vorhergehenden Ansprüchen, **dadurch gekennzeichnet, daß** diese therapeutisch bei dermalen oder transdermalen Systemen eingesetzt werden, die als pharmazeutische Formulierung halbfeste oder flüssige Formen darstellen, insbesondere Salben, Gele, Cremes, Lotionen, Emulsionen, Suspensionen oder Lösungen.

14. Vorrichtungen nach vorhergehenden Ansprüchen, **dadurch gekennzeichnet, daß** diese zur beschleunigten Desintegration epidermaler oder dermaler Wirkstoffdepots eingesetzt werden,_insbesondere Depots mit den Hormonen Insulin, Wachtumshormon, Estradiol, Progesteron, Testosteron, einschließlich ihrer chemischen Analoga.

15. Vorrichtungen nach vorhergehenden Ansprüchen, **dadurch gekennzeichnet, daß** diese als pflasterartige dermale oder transdermale Diagnosesysteme zur Gewinnung und Analyse der natürlichen Flüssigkeit der Haut, Schweiss und dermale Interstitialflüssigkeit, eingesetzt sind, inbesondere zur Analyse der in diesen enthaltenen Substanzen Glukose, Laktat, Elektrolyten, Adrenalin, Kreatinin, Alkohol, sowie von Arzneimitteln und Drogen.

16. Vorrichtungen nach vorhergehenden Ansprüchen, **dadurch gekennzeichnet, daß** diese als pflasterartige dermale oder transdermale nicht-invasive Diagnosesysteme eingesetzt sind, wobei Sammlung und Analyse der auf die Hautoberfläche austretenden Hautflüssigkeit mittels in diese integrierter Sammel- und Sensor-Vorrichtungen erfolgt, um die der thermodynamische Aktor zirkulär angeordnet ist, wobei die Hautflüssigkeit durch eine mit kapillaren Kanälen ausgestattete, plattenartige Sammelvorrichtung aufgenommen wird und die Flüssigkeit über mit dieser in Verbindung stehende elektronische Chemosensoren oder chemische Teststreifen analysiert und ausgewertet wird, und wobei dies zur nicht-invasiven Analyse von insbesondere Glukose, Laktat, Elektrolyten, Adrenalin, Kreatinin, Arzneimitteln, Alkohol und Drogen eingesetzt wird.

17. Vorrichtungen nach vorhergehenden Ansprüchen, **dadurch gekennzeichnet, daß** diese als pflasterartige dermale oder transdermale mikro-invasive Diagnosesysteme eingesetzt sind, wobei die Sammlung und Analyse interstitieller Hautflüssigkeit mittels einer integrierten Sammel- und Sensor-Vorrichtung erfolgt um die der thermodynamische Aktor zirkulär angeordnet ist, wobei die interstitielle Hautflüssigkeit durch eine mit Mikrokanülen ausgestattete, plattenartige Sammelvorrichtung, die geeignet ist die oberste epidermale Hautlage zu durchdringen, aufgenommen oder kontaktiert wird und die Flüssigkeit über mit dieser in Verbindung stehende elektronische Chemosensoren oder chemische Teststreifen analysiert und ausgewertet wird, und wobei dies zur mikro-invasiven Analyse von insbesondere Glukose, Laktat, Elektrolyten, Adrenalin, Kreatinin, Arzneimitteln und Drogen eingesetzt wird.

18. Vorrichtungen nach vorhergehenden Ansprüchen, **dadurch gekennzeichnet, daß** diese als pflasterartige dermale oder transdermale nicht-invasive Diagnosesysteme eingesetzt sind wobei, die Sammel- und Leitvorrichtung für Hautflüssigkeit, insgesamt oder in Teilen, aus polymeren Hohlfasern, aus Mikrokanülen oder Hohlsonden aus metallischem, polymerem oder keramischem Material sind, wobei deren Anstellwinkel zur Perforation der Haut senkrecht, schräg oder tangential justiert sein kann, und wobei der Anstellwinkel auch durch weitere Vorrichtungen reversibel umjustiert werden kann.

19. Vorrichtungen nach vorhergehenden Ansprüchen, **dadurch gekennzeichnet, daß** diese als pflasterartige dermale oder transdermale nicht-invasive oder mikro-invasive Diagnose-Systeme eingesetzt sind, wobei die integrierten Sensor-Vorrichtungen als planare elektronische Chemosensoren ausgestaltet sind.

20. Vorrichtungen nach vorhergehenden Ansprüchen, **dadurch gekennzeichnet, daß** diese als pflasterartige dermale oder transdermale nicht-invasive oder mikro-invasive Diagnosesysteme eingesetzt sind, bei denen die Sensorvorrichtungen in diese reversibel eingeschoben und entnommen werden können.

21. Vorrichtungen nach vorhergehenden Ansprüchen, **dadurch gekennzeichnet, daß** diese als pflasterartige dermale oder transdermale nicht-invasive oder mikro-invasive Diagnosesysteme eingesetzt sind, bei denen die Analyse der Flüssigkeit mittels in diese reversibel einschiebbarer und entnehmbarer chemischer Testreifen erfolgt.

22. Vorrichtungen nach vorhergehenden Ansprüchen, **dadurch gekennzeichnet, daß** diese auch im Tierbereich eingesetzt werden.

EP 1 426 024 A1

**Abbildung 1**

**Abbildung 2**

**Abbildung 3**

**Abbildung 4**

EP 1 426 024 A1

**Abbildung 5**

**Abbildung 6**

**Abbildung 7**

**Abbildung 8**

**Abbildung 9**

**Abbildung 10**

**Europäisches Patentamt**

## EUROPÄISCHER RECHERCHENBERICHT

Nummer der Anmeldung

EP 03 02 3041

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 6 419 651 B1 (AUGUSTINE SCOTT D) 16. Juli 2002 (2002-07-16) | 1,3-5, 8-15,22 | A61F7/04 |
| Y | * Spalte 3 - Spalte 4; Abbildung 20 * | 2,6,7, 16-21 | |
| | * abstract * * Spalte 12 - Spalte 17 * --- | | |
| X | DE 101 02 817 A (LOHMANN THERAPIE SYST LTS) 1. August 2002 (2002-08-01) * Spalte 5 - Spalte 8; Abbildung 3 * --- | 1,3-5, 8-15,22 | |
| Y | US 4 279 255 A (HOFFMAN JOHN C) 21. Juli 1981 (1981-07-21) * abstract * * Abbildungen 1,3,4 * --- | 2,7 | |
| Y | US 5 036 861 A (ANDERSON CARTER R ET AL) 6. August 1991 (1991-08-06) * abstract * * Spalte 1; Abbildungen 1,7 * --- | 16-21 | |
| Y | WO 02 28454 A (INSULET CORP) 11. April 2002 (2002-04-11) * abstract * ----- | 6 | **RECHERCHIERTE SACHGEBIETE (Int.Cl.7)** A61F A61K |

Der vorliegende Recherchenbericht wurde für alle Patentansprüche erstellt

| Recherchenort | Abschlußdatum der Recherche | Prüfer |
|---|---|---|
| MÜNCHEN | 5. Dezember 2003 | Edward, V |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes Dokument

EPO FORM 1503 03.82 (P04C03)

**EP 1 426 024 A1**

**ANHANG ZUM EUROPÄISCHEN RECHERCHENBERICHT**
**ÜBER DIE EUROPÄISCHE PATENTANMELDUNG NR.**          EP 03 02 3041

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-12-2003

| Im Recherchenbericht angeführtes Patentdokument | | Datum der Veröffentlichung | Mitglied(er) der Patentfamilie | | Datum der Veröffentlichung |
|---|---|---|---|---|---|
| US 6419651 | B1 | 16-07-2002 | US | 6264622 B1 | 24-07-2001 |
| | | | US | 5986163 A | 16-11-1999 |
| | | | AT | 198135 T | 15-01-2001 |
| | | | AU | 719219 B2 | 04-05-2000 |
| | | | AU | 5256498 A | 07-08-1998 |
| | | | CN | 1247462 A | 15-03-2000 |
| | | | DE | 69703748 D1 | 25-01-2001 |
| | | | DE | 69703748 T2 | 02-08-2001 |
| | | | EP | 0954261 A1 | 10-11-1999 |
| | | | JP | 2001509058 T | 10-07-2001 |
| | | | WO | 9831310 A1 | 23-07-1998 |
| | | | US | 2003144619 A1 | 31-07-2003 |
| | | | US | 6045518 A | 04-04-2000 |
| | | | US | 6217535 B1 | 17-04-2001 |
| | | | US | 6406448 B1 | 18-06-2002 |
| | | | AT | 175572 T | 15-01-1999 |
| | | | CA | 2138402 A1 | 06-01-1994 |
| | | | DE | 69323080 D1 | 25-02-1999 |
| | | | DE | 69323080 T2 | 27-05-1999 |
| | | | DK | 645995 T3 | 30-08-1999 |
| | | | EP | 0645995 A1 | 05-04-1995 |
| | | | EP | 0745365 A2 | 04-12-1996 |
| | | | EP | 0745366 A2 | 04-12-1996 |
| | | | ES | 2125993 T3 | 16-03-1999 |
| | | | GR | 3029560 T3 | 30-06-1999 |
| | | | HK | 1010330 A1 | 24-03-2000 |
| | | | WO | 9400090 A2 | 06-01-1994 |
| | | | US | 2002183676 A1 | 05-12-2002 |
| | | | US | 2003167029 A1 | 04-09-2003 |
| | | | US | 6113561 A | 05-09-2000 |
| | | | US | 6071254 A | 06-06-2000 |
| | | | US | 5961480 A | 05-10-1999 |
| | | | US | 6241697 B1 | 05-06-2001 |
| | | | US | 6213966 B1 | 10-04-2001 |
| | | | US | 6241698 B1 | 05-06-2001 |
| | | | US | 6465708 B1 | 15-10-2002 |
| | | | US | 6423018 B1 | 23-07-2002 |
| | | | US | 5964723 A | 12-10-1999 |
| | | | US | 5954680 A | 21-09-1999 |
| DE 10102817 | A | 01-08-2002 | DE | 10102817 A1 | 01-08-2002 |
| | | | WO | 02058678 A2 | 01-08-2002 |
| | | | EP | 1353654 A2 | 22-10-2003 |
| US 4279255 | A | 21-07-1981 | KEINE | | |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT
## ÜBER DIE EUROPÄISCHE PATENTANMELDUNG NR.

EP 03 02 3041

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-12-2003

| Im Recherchenbericht angeführtes Patentdokument | | Datum der Veröffentlichung | Mitglied(er) der Patentfamilie | | Datum der Veröffentlichung |
|---|---|---|---|---|---|
| US 5036861 | A | 06-08-1991 | KEINE | | |
| WO 0228454 | A | 11-04-2002 | AU | 9658801 A | 15-04-2002 |
| | | | CA | 2423717 A1 | 11-04-2002 |
| | | | EP | 1332440 A2 | 06-08-2003 |
| | | | WO | 0228454 A2 | 11-04-2002 |
| | | | US | 2002040208 A1 | 04-04-2002 |

EPO FORM P0461

Für nähere Einzelheiten zu diesem Anhang : siehe Amtsblatt des Europäischen Patentamts, Nr.12/82